# EUROPEAN PATENT SPECIFICATION

(11) **EP 2 086 999 B1**
(45) Date of publication and mention of the grant of the patent: **09.01.2013**
(21) Application number: 07816718.6
(22) Date of filing: 01.11.2007
(51) Int. Cl.: C07K 14/47, C07D 519/00, C07D 231/18, C07D 237/26, C07D 209/14, A61K 31/437, A61K 31/415, A61K 31/517, A61K 31/403, A61P 35/00, G01N 33/68, C07D 471/04, C07D 333/70

(54) **INHIBITORS FOR DISRUPTING THE INTERACTION OF UBIQUITINATION RELATED ENZYMES AND USES THEREOF**
INHIBITOREN ZUM STÖREN DER WECHSELWIRKUNG VON MIT DER UBIQUITINIERUNG IM ZUSAMMENHANG STEHENDEN ENZYMEN UND ANWENDUNGEN DAVON
INHIBITEURS POUR INTERROMPRE L'INTERACTION D'ENZYMES ASSOCIÉES À L'UBIQUITINYLATION ET LEURS UTILISATIONS

(30) Priority: 02.11.2006 US 856125 P
(43) Date of publication of application: 12.08.2009
(73) Proprietor: Staidson (Beijing) Biopharmaceuticals Co., Ltd, Beijing 100176 (CN)
(72) Inventor: Huang, Lan, Bronx, NY 10463 (US); Li, Xing, Chesterfield, MO 63017 (US)
(74) Representative: Moinas & Savoye SA
(86) International application number: PCT/CN2007/003105
(87) International publication number: WO 2008/052441

(56) References cited:
- WO-A1-2005/037845
- WO-A1-2006/002284
- WO-A1-2006/074262
- WO-A2-2005/000876
- WONG BRIAN R ET AL: "Drug discovery in the ubiquitin regulatory pathway." DRUG DISCOVERY TODAY, vol. 8, no. 16, 15 August 2003 (2003-08-15), pages 746-754, XP002585137 ISSN: 1359-6446
- SWINNEY DAVID C ET AL: "A small molecule ubiquitination inhibitor blocks NF-kappaB-dependent cytokine expression in cells and rats" JOURNAL OF BIOLOGICAL CHEMISTRY, vol. 277, no. 26, 28 June 2002 (2002-06-28), pages 23573-23581, XP002585138 ISSN: 0021-9258
- PAPADOPOULOS, ELEFTHERIOS P. ET AL: "Reactions of azoles with isocyanates at elevated temperature" 1979, JOURNAL OF ORGANIC CHEMISTRY , 44(1), 99-104 CODEN: JOCEAH; ISSN: 0022-3263 , XP002585139 * page 104; table III; compound 13d *
- RIED W ET AL: "Structure determination of N<6>-, 9- and 7-acyladenines by <1>H- and <13>C-NMR spectroscopy of solids and in solution" HELVETICA CHIMICA ACTA 1989 CH, vol. 72, no. 7, 1989, pages 1597-1607, XP002585140 ISSN: 0018-019X
- DATABASE CA [Online] CHEMICAL ABSTRACTS SERVICE, COLUMBUS, OHIO, US; 1978, TERASHIMA, MASANAO ET AL: "Photochemistry of the amide system. VIII. Photoinduced reactions. XXXIV. Photoarylation. I. Photochemical synthesis of benzothieno[2,3-c][1,x]diazanaphthalene systems by intramolecular dehydrochlorination" XP002585141 retrieved from STN Database accession no. 1978:62314
- HALVERSON, AILEEN PFLEIDER ET AL: "Synthesis and total 1H-NMR assignment of [1]benzothieno[2,3-c][1,10]phenanthroline" JOURNAL OF HETEROCYCLIC CHEMISTRY , 33(3), 727-730 CODEN: JHTCAD; ISSN: 0022-152X, 1996, XP002585142
- DATABASE REGISTRY [Online] CHEMICAL ABSTRACTS SERVICE, COLUMBUS, OHIO, US; 9 August 2006 (2006-08-09), XP002585143 retrieved from stn Database accession no. 900004-38-8
- DATABASE REGISTRY [Online] CHEMICAL ABSTRACTS SERVICE, COLUMBUS, OHIO, US; 8 November 2006 (2006-11-08), XP002585144 retrieved from stn Database accession no. 899734-49-7
- DATABASE REGISTRY [Online] 17 March 2006 (2006-03-17), XP002585145 retrieved from stn Database accession no. 877155-98-1
- DATABASE REGISTRY [Online] CHEMICAL ABSTRACTS SERVICE, COLUMBUS, OHIO, US; 12 August 2002 (2002-08-12), XP002585146 retrieved from stn Database accession no. 443645-90-7
- DATABASE REGISTRY [Online] CHEMICAL ABSTRACTS SERVICE, COLUMBUS, OHIO, US; 14 August 2001 (2001-08-14), XP002585147 retrieved from stn Database accession no. 351330-04-6
- DATABASE REGISTRY [Online] CHEMICAL ABSTRACTS SERVICE, COLUMBUS, OHIO, US; 25 April 2001 (2001-04-25), XP002585148 retrieved from stn Database accession no. 332382-01-1
- DATABASE REGISTRY [Online] CHEMICAL ABSTRACTS SERVICE, COLUMBUS, OHIO, US; 24 April 2001 (2001-04-24), XP002585149 retrieved from stn Database accession no. 332156-40-8
- DATABASE REGISTRY [Online] CHEMICAL ABSTRACTS SERVICE, COLUMBUS, OHIO, US; 20 April 2001 (2001-04-20), XP002585150 retrieved from stn Database accession no. 331948-62-0
- DATABASE REGISTRY [Online] CHEMICAL ABSTRACTS SERVICE, COLUMBUS, OHIO, US; 16 January 2001 (2001-01-16), XP002585151 retrieved from stn Database accession no. 314045-90-4
- DATABASE REGISTRY [Online] CHEMICAL ABSTRACTS SERVICE, COLUMBUS, OHIO, US; 15 January 2001 (2001-01-15), XP002585152 retrieved from stn Database accession no. 313966-53-9
- DATABASE REGISTRY [Online] CHEMICAL ABSTRACTS SERVICE, COLUMBUS, OHIO, US; 21 November 2000 (2000-11-21), XP002585153 retrieved from stn Database accession no. 303794-05-0
- ZHENG NING ET AL: "Structure of a c-Cbl-UbcH7 complex: RING domain function in ubiquitin-protein ligases" CELL, vol. 102, no. 4, 18 August 2000 (2000-08-18), pages 533-539, XP002585154 ISSN: 0092-8674
- HUANG LAN ET AL: "Structure of an E6AP-UbcH7 complex: Insights into ubiquitination by the E2-E3 enzyme cascade" SCIENCE (WASHINGTON D C), vol. 286, no. 5443, 12 November 1999 (1999-11-12), pages 1321-1326, XP002585155 ISSN: 0036-8075
- DATABASE GENBANK [Online] 17 October 2006 HUANG L. ET AL.: 'Structure of an E6AP-UbcH7 complex: insights into ubiquitination by the E2-E3 enzyme cascade', XP008115340 Database accession no. (Q05086) & SCIENCE vol. 286, 1999, pages 1321 - 1326
- HUIBREGTSE J.M. ET AL.: 'Cloning and expression of the cDNA for E6-AP, a protein that mediates the interaction of the human papillomavirus E6 oncoprotein with p53' MOLECULAR AND CELLULAR BIOLOGY vol. 13, no. 2, February 1993, pages 775 - 784, XP000602210

## Description

### Field of the Invention

The present invention relates to structure-based drug design in ubiquitination pathways and using these chemical compounds for treatment of cancer. In particular, the present invention relates to the identification of these classes of inhibitors of E2-E3, a highly regulated protein-protein surface interaction. The compositions of these classes of compounds and their analogs are protected.

The invention is applicable to cancers and other diseases general in mammals. The reference to human biochemistry is not intended to be limiting, but illustrative. The term patient or body or reference to humans is utilized for convenience, but includes all mammalian patients or bodies.

### Background of the Invention

### Introduction

Ubiquitin-mediated degradation is a protein regulatory mechanism that controls protein abundance at a post-translational level. It is as significant a control mechanism as phosphorylation. This pathway plays important roles in DNA repair, cell cycle control, class I antigen processing, chromosomal organization, signal transduction, receptor mediated endocytosis, and apoptosis (Hochstrasser, M. 1996). Mis-regulation of these processes can cause uncontrolled cell growth, or cancer, including colon cancer, breast cancer, lung cancer, leukemia, lymphoma, and myeloma (Konstantinopoulos PA. et al.; 2006starita LM et al; Montagut C. et al.; Pulczynski, S; Keating, M.J.; Hall, E.J.; Magrath, I.; Voutsadakis, I.A.).

The ubiquitination cascade involves the successive action of E1, E2 and E3 activities (Scheffner, M. et al, 1998). The E1 (ubiquitin-activating) enzyme, in an ATP-dependent reaction, activates ubiquitin by forming a thioester bond between its active site cysteine and the carboxyl-terminus of ubiquitin. Ubiquitin is then transferred to the active site cysteine of E2 (ubiquitin-conjugating) enzymes, maintaining a thioester linkage. E3 ubiquitin-protein ligases are minimally defined as additional proteins or protein complexes necessary for the recognition and ubiquitination of specific substrates. E3 mediates the transfer of ubiquitin from the E2 to the lysine side chains, first on the substrate, subsequently on ubiquitin. Finally, the poly-ubiquitinated substrates are recognized by the proteasome for degradation. There are two classes of E3: the RING finger E3 family, which have not been shown to form thioester linkage with ubiquitin (Scheffner, M. et al).; and the Hect domain E3 ligase family (Schwarz, S.E. et al.). Hect domain stands for homologous to E6-associated protein [E6AP] carboxyl-terminal domain. This Hect class of E3 proteins forms ubiquitin thioester intermediates and promotes poly-ubiquitination of the substrates.

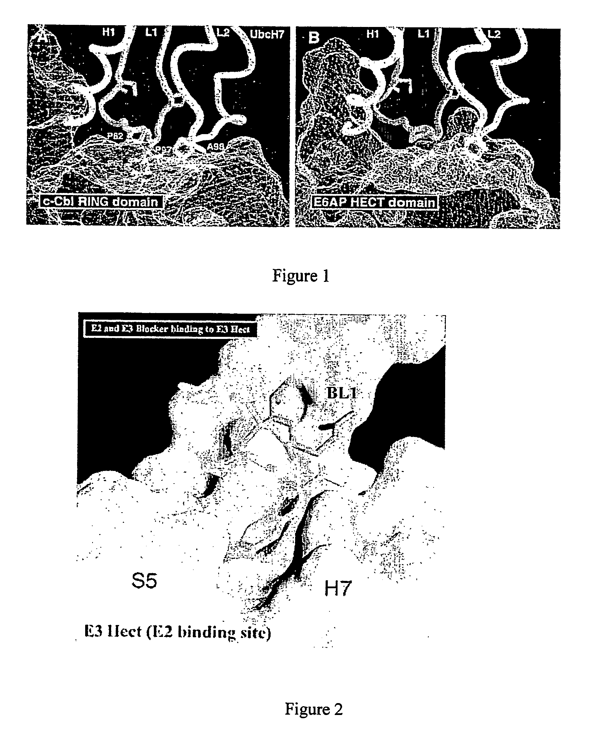

Both RING finger E3 and HECT E3 interact with E2 (all E2 displays the same structure) in similar structural manner (Huang, L et al, Zheng, N. et al.) But there are subtle differences in the binding pocket (seen in Fig. 1). Thus inhibitors of this surface could affect the ubiquitination of substrate of both classes of E3 ligases with selectivity. These inhibitors will have many applications in a variety of diseases. It is very difficult to disrupt protein-protein interaction. However, this E2-E3 case is quite unique. According to Inventor's biochemical studies, the binding between E2 (UbcH7) and E3 (E6AP's Hect domain) was quite weak, not withstanding the gel filtration force. The binding could only be detected in native gel electrophoresis and co-crystallization. Furthermore, conducted by Inventor, thorough analysis of the interface of the E2-E3 complexes revealed a well-defined hydrophobic pocket that appeared critical for the coupling interactions of the E2-E3 complex. According to the size, shape and nature of the hydrophobic pocket the binding is best suited for a small molecule target. Thus the interaction point between E2 and E3 could easily be disrupted by small molecules, which forms a basis for this invention.

One famous substrate for ubiquitination pathways is the "genome guardian" tumor suppressor P53. The inactivation of the tumor suppressor p53 has the highest incidence in cancer. Following exposure to genotoxic agents, activation of the transcription factor p53 prevents replication of damaged DNA by either instituting cell cycle arrest at G1/S checkpoint or by targeting the damaged cells for apoptosis (Oren, M.). Inactivation of p53 occurs in more than 50% of all human cancers. For example, p53 mis-regulation is involved in 35% of Burkitt's lymphoma and 60% of L3 type B-cell acute lymphoblastic leukemia (Soussi, T. & Jonveaux, P.). In addition to p53 mutation, ubiquitin-mediated degradation of p53 is one of the efficient ways to inactivate p53, and it frequently occurs in leukemia and lymphoma (Masdehors, P. et al.; Bueso-Ramos, C.E. et al.; Teoh, G. et al.; Pan, Y. & Haines, D.S.; Gustafsson, B. & Stal, O.). P53 degradation is mediated through both classes of E3 ligases in different cellular context. In normal cells, a RING finger E3 ligase MDM2 regulates p53 level by degradation (Fuchs, S.Y. et al; Honda, R.). In human papillomavirus (HPV) infected cells, E6AP, "hijacked" by viral protein E6, ubiquitinates p53. Thus inhibition of the degradation of tumor suppressor P53 through blocking the E2-E3 binding and thus ubiquitin transfer to P53 could potentially inhibitor tumor growth.

Other diseases, such as diabetes and neuro-degenerative diseases are implicated to be caused by mal-function of ubiquitination pathways. Inappropriate degradation of insulin signaling molecules such as insulin receptor substrates (IRS-1 and IRS-2) has been demonstrated in experimental diabetes (Balasubramanyam M. et al.) A series of recent reports have evaluated the pre-clinical efficacy of the proteasome inhibitor MLN519 for the treatment of focal ischemic/reperfusion brain injury in rats (Williams AJ, et al.).

The present invention provides novel chemical compound classes that were discovered using structure-based drug design techniques, based on ubiquitination cascade E2-E3 complex crystal structures. E2 and E3 are important and necessary enzymes for protein degradation. Malfunction of protein degradation cause many serious diseases. Protein ubiquitination is as important as protein phosphorylation. Thus the importance of E2 and E3 enzymes rivals that of kinases, which inhibitors are developed as novel drugs on the market. We also claim our classes of novel compounds and their analogs useful for the treatment of cancer, such as breast cancer, colon cancer, cervical cancer, lung cancer, liver cancer, and brain tumor.

### Summary of the Invention

One object of this invention is to define the specific pocket of the E2-E3 binding interface that is suitable for binding of a small therapeutic agent, thus effective at disrupting the E2-E3 complex formation leading to the therapeutic effect controlled by the E2-E3 signaling pathway (seen in Fig. 1 and 2).

Another object of the present invention is to provide processes, which were used to identify compound classes that fit into E3's E2 binding pocket.

Another object of the present invention is to provide the compositions of these classes of compounds.

An additional object of the invention is to show these classes of compounds useful in inhibition of cancer cell growth, and thus will be useful in the treatment of subjects suffering from cancer comprising these classes of compounds or analogs thereof alone, or in combination with other chemotherapy agents or pharmaceutical carriers.

Therefore, on one aspect, the present invention provides a E2-E3 inhibitor with anti-tumor activity for use in therapy, which is a compound having the following formula I : 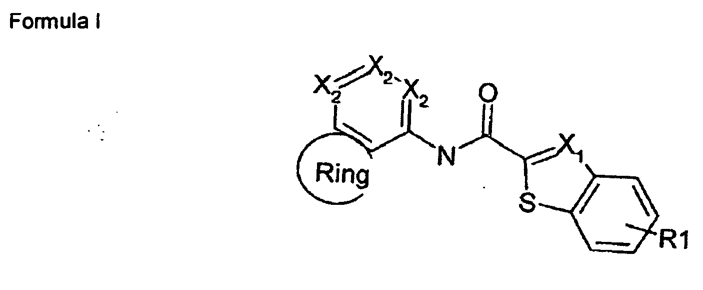 wherein
X1 = N, C
R1 = halogens (F,Cl,Br), Me, OMe, OH
mono- or multi- substituted at the free valences using combinations of the functional groups

X2 = one of more of it could be either C or N
Ring = five- or six- membered rings fuzed with the aromatic ring explicitly drawn,
including benzene, pyridine, pyrimidine, pyrizine, pyrrole, imidazole, furan, oxazole

In a second aspect, the present invention provides a pharmaceutical composition comprising at least one of the compounds as stated above and a pharmaceutical carrier.

In a third aspect, the present invention provides a use of the inhibitor of as stated above in the manufacture of a medicament for the treatment of cancer. Preferably, the cancer is brain tumor, lung cancer, ovarian cancer, bladder cancer, cervical cancer, colon cancer, breast cancer, and prostate cancer.

In a fourth aspect of the present invention, the use of the inhibitor involve the administration of a therapeutically effective amount of at least one of the compounds as stated above to a subject in need thereof.

In a fifth aspect, the present invention provides the use of the inhibitor of formula I for the manufacture of an imaging agent for tumor diagnosis.

### Description of the Figures

FIG. 1 illustrates the structure comparison of RING finger E3 and Hect E3's E2 binding domains to E2, with similarities and differences.
FIG. 2 illustrates the zoomed in view of E2 (specific residues contacting E3) and E3's E2 binding pocket.
FIG 3 illustrates the IH NMR data of synthesized compound Mol 11 (invention).
FIG. 4 illustrates the IHNMR data of synthesized compound Mol 21 (comparative).
FIG. 5 illustrates the IH NMR data of synthesized compound Mol 44 (comparative).
FIG. 6 illustrates the IHNMR data of synthesized compound Mol 640 (comparative).

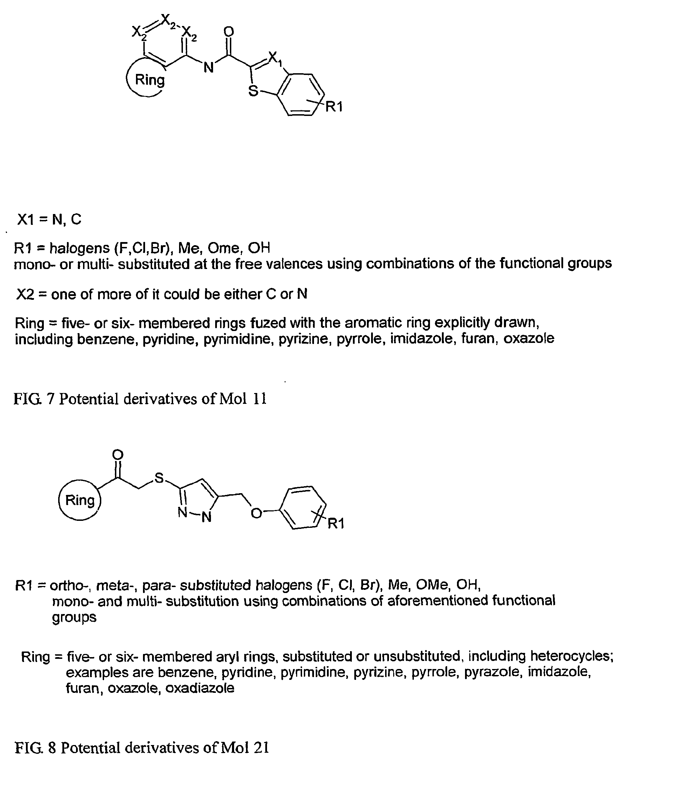

FIG. 7 describes the potential derivatives of Mol 11 (invention).
FIG. 8 describes the potential derivatives of Mol 21 (comparative).

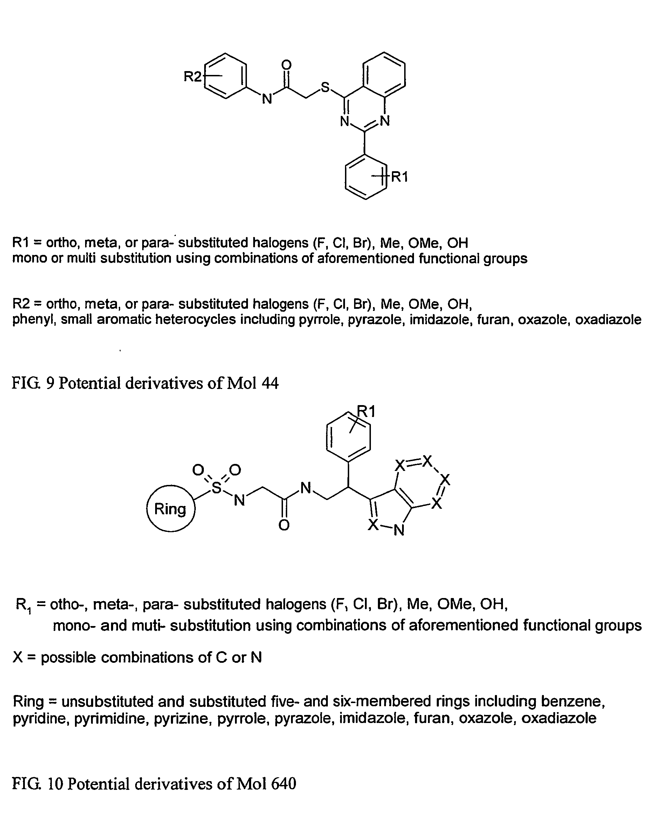

FIG. 9 describes the potential derivatives of Mol 44 (comparative).
FIG 10 describes the potential derivatives of Mol 640 (comparative).

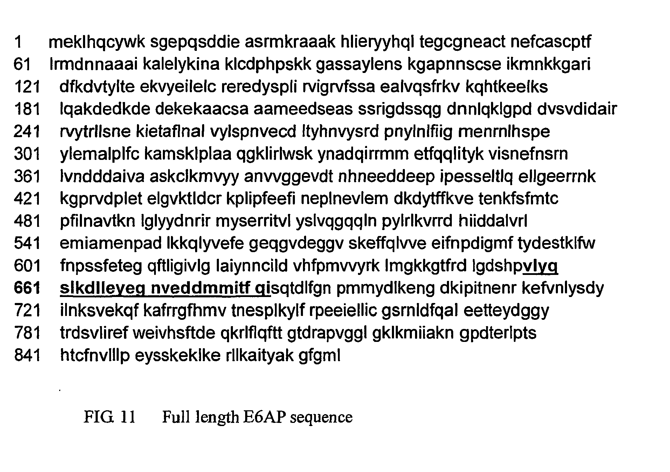

FIG 11 describes the full length sequence of E6AP from NCBI database. The region of the interest pocket is underlined.

### Detailed Description of the Invention

### Definition

"E2 inhibitors" in the context means "inhibitors which disrupts E2 and E3 interaction".

### 1) Identification of critical binding site for small-molecule inhibitors

Protein-protein interactions are universally deemed difficult to disrupt by a small molecular agent. However, through mapping of E2-E3 interaction and molecular surface analysis of the E2-E3 interface, we've identified a binding site on E3 that appear critical for E2-E3 complex formation and suitable for a small molecule interaction. This pocket resides on E3, and is
occupied by Phe63 of E2. The composing residues (from E3) of this binding site are: Val657, Leu658, Ser661, Leu662, Leu665, Met 676, Ile678, Ile682, Ile705, Phe713, Tyr717.

Lipophilic potential analysis reveals that this pocket is highly hydrophobic, with contributions from hydrophbic side chains of Leu658, Leu662, Leu665, Met 676, Ile678, Ile682, Ile705, Phe713, Tyr717 of E3.

By targeting this small hydrophobic binding pocket we will demonstrate that sufficient potency to disrupt the E2-E3 signaling cascade and consequently the ubiquitination pathway could be achieved by a small molecular agent of molecular weight around five hundred, embodying all favorable properties of a pharmaceutical agent including potency, stability (both in vitro and in vivo), efficacy and safety.

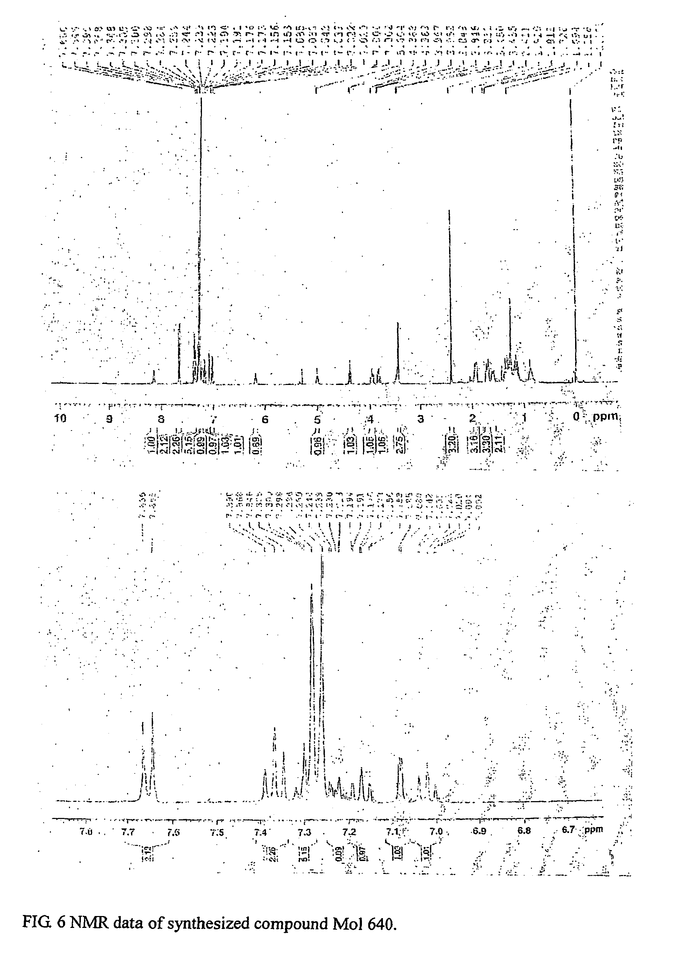

While potency could be primarily achieved by occupying the small hydrophobic pocket, we also disclose that selectivity against unwanted targets could be achieved by extending the interaction at the E2-E3 interface into the secondary binding pocket illustrated in Figure 6. The secondary binding pocket situates adjacent to the primary hydrophobic pocket, and is comparatively less hydrophobic. It is most suited for binding of polar chemical moieties and/or combinations of heterocycles. Hydrogen bond interactions could be engaged with specific residues in the secondary pocket to further enhance potency and selectivity. It is demonstrated that by designing the optimal linkers between the two binding pockets the complementary occupation of both binding pockets could be achieved by a single chemical entity. Due to the extensive optimization toward the E2-E3 interface, the designed chemical entities are expected to highly selective against other biological targets that it may be exposed to during lifetime of action, therefore minimizing the potential side effects.

### 2) Structure-based drug design

A suite of computational technologies was engaged in characterizing the interactions between E2 and E3, including protein surface generation and comparison, hydrogen bond analysis, property mappings including hydrophobicity and electrostatic potentials and overall shape complementarities at the interface of the binary complex. A small, well-defined hydrophobic pocket was discovered at the E2-E3 interface by this study, which appeared critical for the coupling interactions of the E2-E3 complex. Therefore targeting the hydrophobic pocket has the promise of disrupting the E2-E3 complex formation, leading to the intervention of the ubiquitination pathway and the subsequent therapeutic effects aforementioned.

Design of suitable inhibitors that bind into the hydrophobic pocket entailed library design, similarity search, virtual screening and de novo design. Several virtual compounds libraries based on the validated synthetic protocols were designed using the commercially available reagents. The virtual compounds were then combined with several commercial vendor collections to generate a larger compound database. The compounds were filtered on a number of druggable properties and the ones that passed all the filtering steps formed the candidate pool, which were subsequently docked into the hydrophobic pocket on E3. A free energy cutoff of -20 kJ/mol yielded several dozens of promising binders, and their binding conformations within the protein environment were visually inspected to ensure the proper binding. This set of compounds were finally optimized inside the binding site by a set of mutational operators coupled with energy evaluations to further optimize their interactions with E3 as well as implant structural novelty into the templates.

### 3) Anti-tumor usage claims.

The invention provides a method of treating cancer in a subject suffering comprising administering the subject a therapeutically effective amount of these classes of compounds or any analogs thereof disclosed herein [hereafter referred to as substance] optionally in combination with a pharmaceutically suitable carrier. The method may be applied where the cancer is a solid tumor or leukemia. In particular, the method is applicable where the cancer is brain tumor, lung cancer, breast cancer, prostate cancer, ovarian cancer, or colorectal cancer.

The subject of the invention also provides a pharmaceutical composition for treating cancer comprising the substance, as an active ingredient, optionally though typically in combination with a pharmaceutically suitable carrier. The pharmaceutical compositions of the present invention may further comprise other therapeutically active ingredients, such as existing chemotherapy agents for combination therapy.

The magnitude of the therapeutic dose of the compounds of the invention will vary with the nature and severity of the condition to be treated and with the particular compound and its route of administration. Any suitable route of administration may be employed for providing a mammal, especially a human, with an effective dosage of a substance disclosed herein. For example, oral, rectal, topical, parenteral, ocular, pulmonary, nasal, etc., routes may be employed. Dosage forms include tablets, troches, dispersions, suspensions, solutions, capsules, creams, ointments, aerosols, etc.

The compositions include compositions suitable for oral, rectal, topical (including transdermal devices, aerosols, creams, ointments, lotions and dusting powders), parenteral (including subcutaneous, intramuscular, intraarterial, and intravenous), ocular (ophthalmic), pulmonary (nasal or buccal inhalation) or nasal administration. Although the most suitable route in any given case will depend largely on the nature and severity of the condition being treated and on the nature of the active ingredient, they may be conveniently presented in unit dosage form and prepared by any of the methods well known in the art of pharmacy.

In preparing oral dosage forms any of the unusual pharmaceutical media may be used, such as water, glycols, oils, alcohols, flavoring agents, preservatives, coloring agents, and the like in the case of oral liquid preparations (e.g., suspensions, elixers and solutions); or carriers such as starches, sugars, microcrystalline cellulose, diluents, granulating agents, lubricants, binders, disintegrating agents, etc., in the case of oral solid preparations are preferred over liquid oral preparations such as powders, capsules and tablets. If desired, capsules may be coated by standard aqueous or non-aqueous techniques. In addition to the dosage forms described above, the compounds of the invention may be administered by controlled release means and devices according to the general knowledge of one skilled in the art.

Pharmaceutical compositions of the present invention suitable for oral administration may be prepared as discrete units such as capsules, cachets or tablets each containing a predetermined amount of the active ingredient in powder or granular form or as a solution or suspension in an aqueous or nonaqueous liquid or in an oil-in-water or water- in-oil emulsion. Such compositions may be prepared by any of the methods known in the art of pharmacy. In general compositions are prepared by uniformly and intimately admixing the active ingredient with liquid carriers, finely divided solid carriers, or both and then, if necessary, shaping the product into the desired form. For example, a tablet may be prepared by compression or molding, optionally with one or more accessory ingredients. Compressed tablets may be prepared by compressing in a suitable machine the active ingredient in a free-flowing form such as powder or granule optionally mixed with a binder, lubricant, inert diluent or surface active or dispersing agent. Molded tablets may be made by molding in a suitable machine, a mixture of the powdered compound moistened with an inert liquid diluent.

The present invention will be better understood from the Experimental Details as following. However, one skilled in the art will understand that the examples described below is only for illustration, and will not limit the protecting scope as defined in the claims.

### EXAMPLES

### Example 1 (invention)

New chemical entity (NCE) Mol 11 was identified from the aforementioned procedures. Its chemical structure is depicted below.

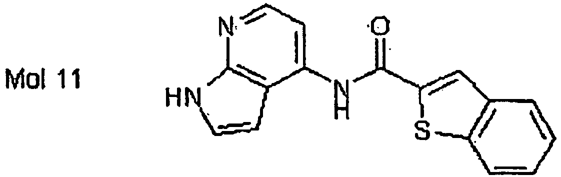

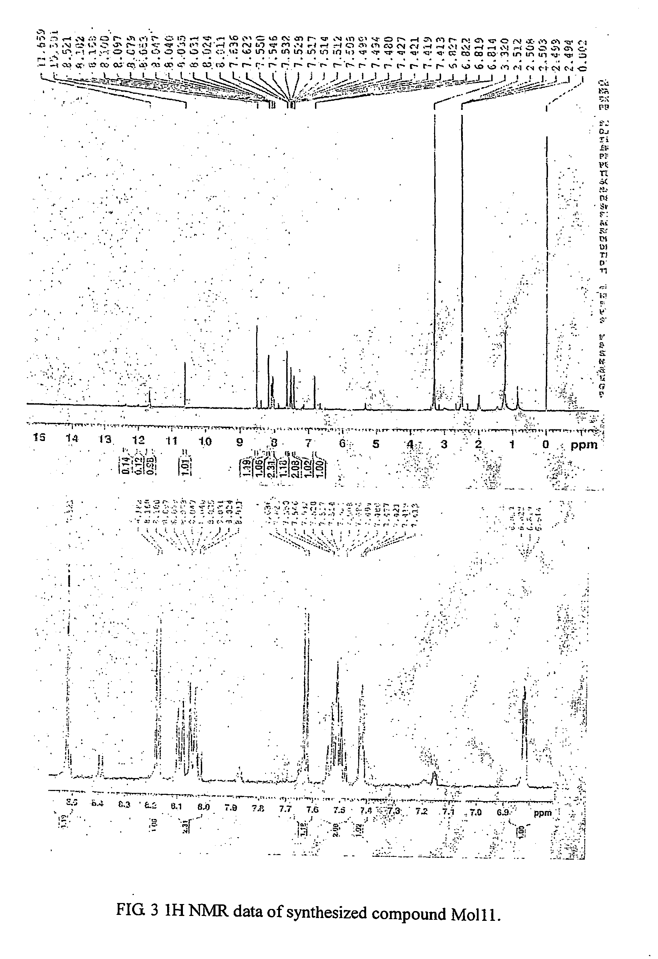

It was synthesized using the route below. Mass spectrometry data confirmed its molecular weight of 294. IH NMR data was recorded, shown in Fig 3.

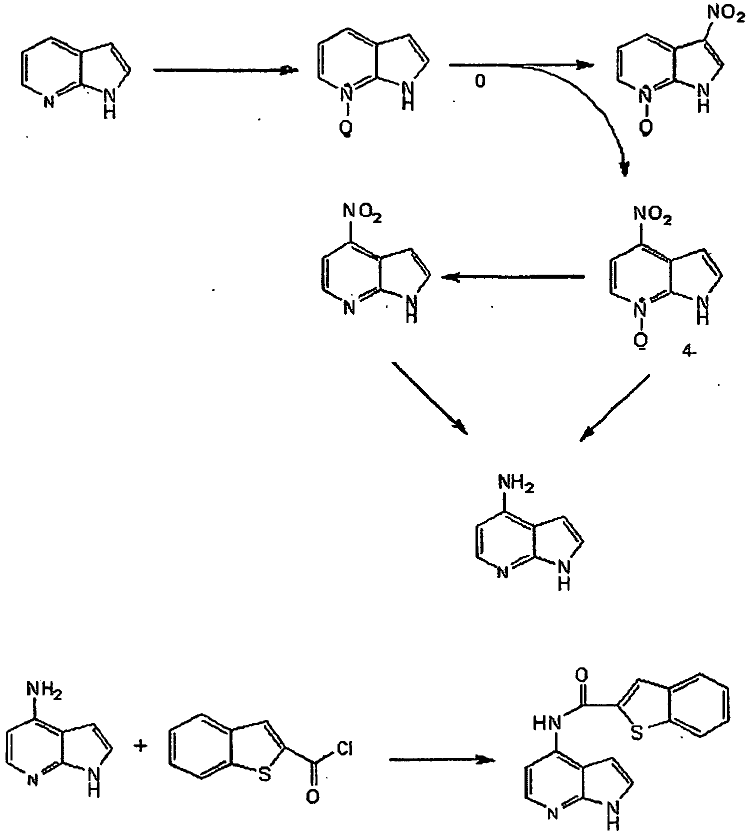

### Example 2 (comparative)

New chemical entity (NCE) was identified from the aforementioned procedures. Its chemical structure is depicted below.

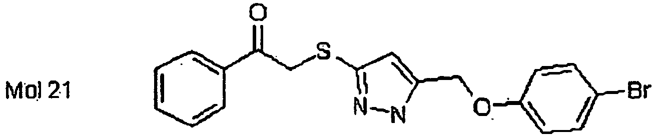

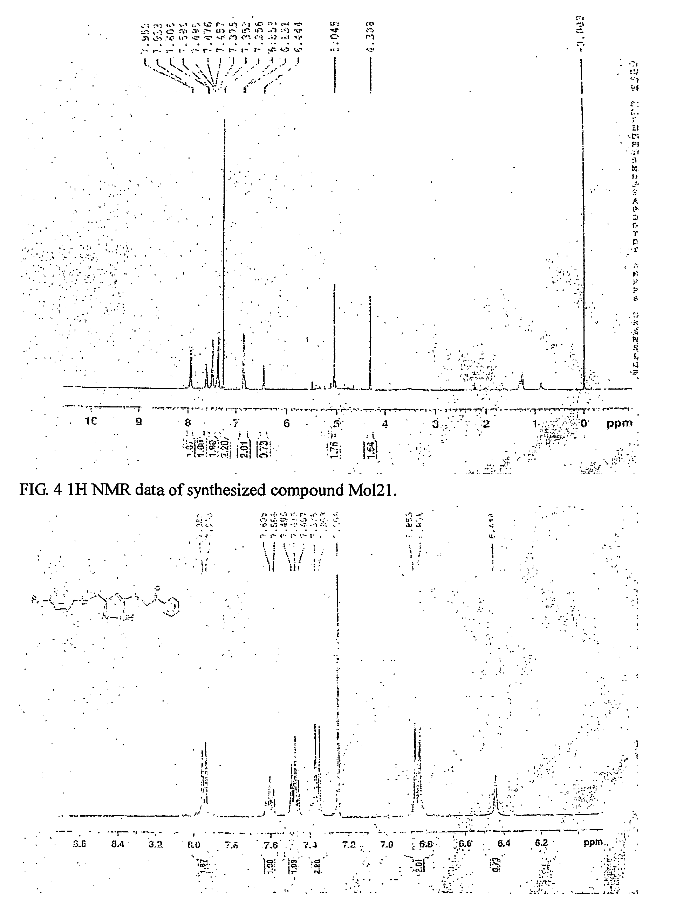

It was synthesized using the route below. Mass spectrometry data confirmed its molecular weight of 403. IH NMR data was recorded, shown in Fig 4.

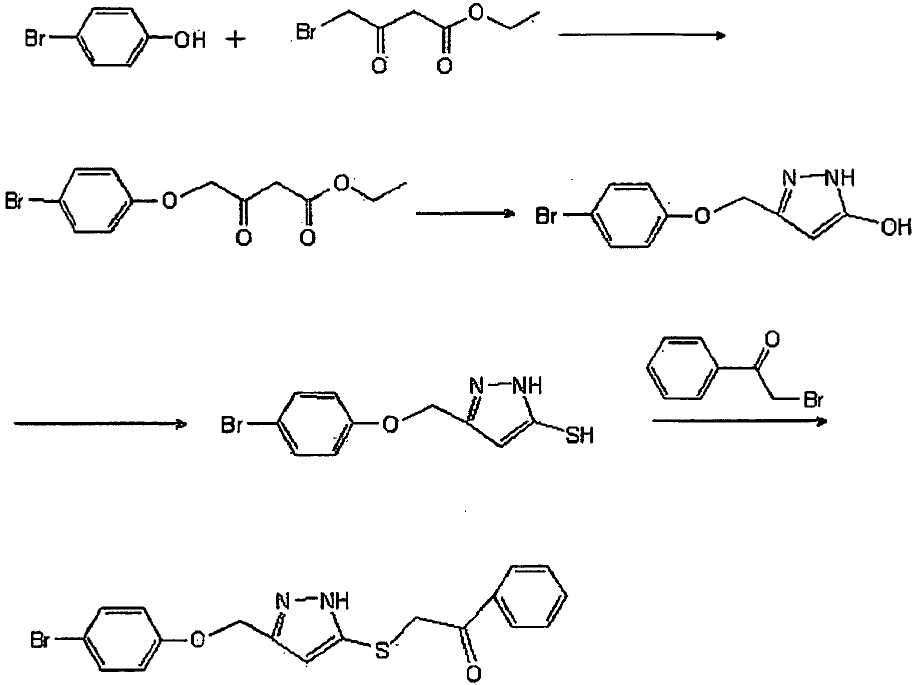

### Example 3 (comparative)

New chemical entity (NCE) Mol 44 was identified from the aforementioned procedures. Its chemical structure is depicted below.

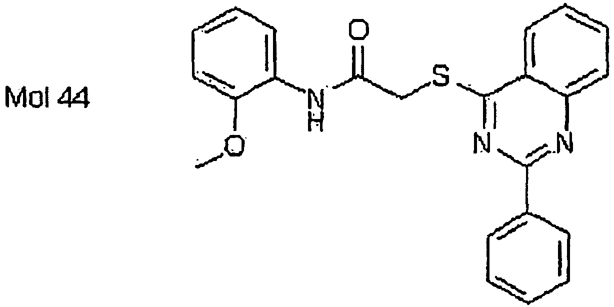

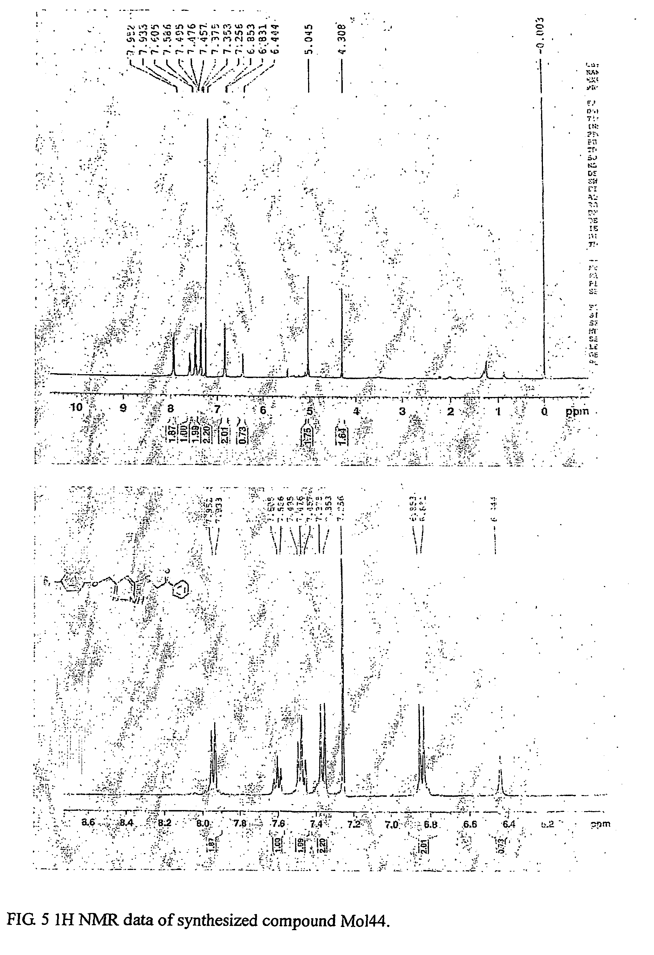

It was synthesized using the route below. Mass spectrometry data confirmed its molecular weight of 402. IH NMR data was recorded, shown in Fig 5.

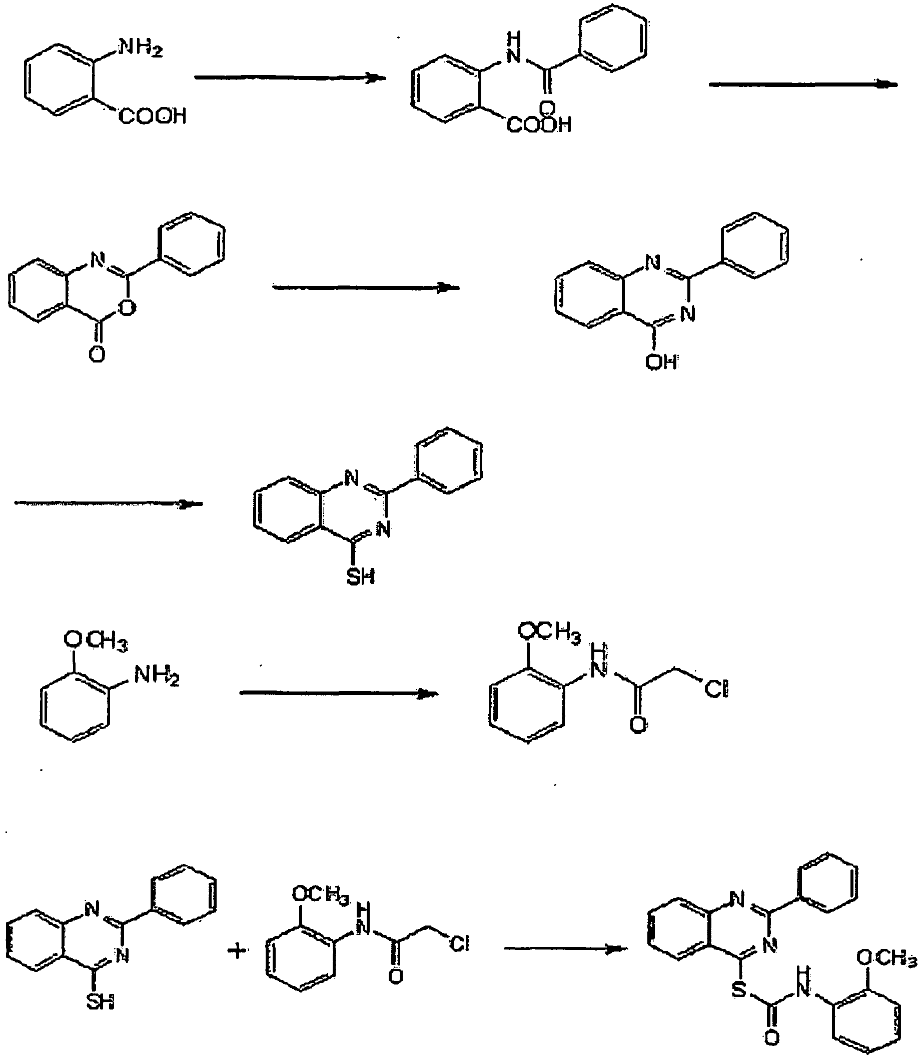

### Example 4 (comparative)

New chemical entity (NCE) Mol 640 was identified from the aforementioned procedures. Its chemical structure is depicted below.

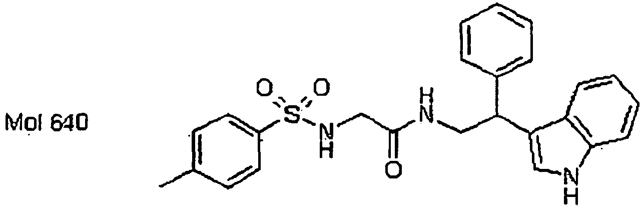

It was synthesized using the route below. Mass spectrometry data confirmed its molecular weight of 448. IH NMR data was recorded, shown in Fig 6.

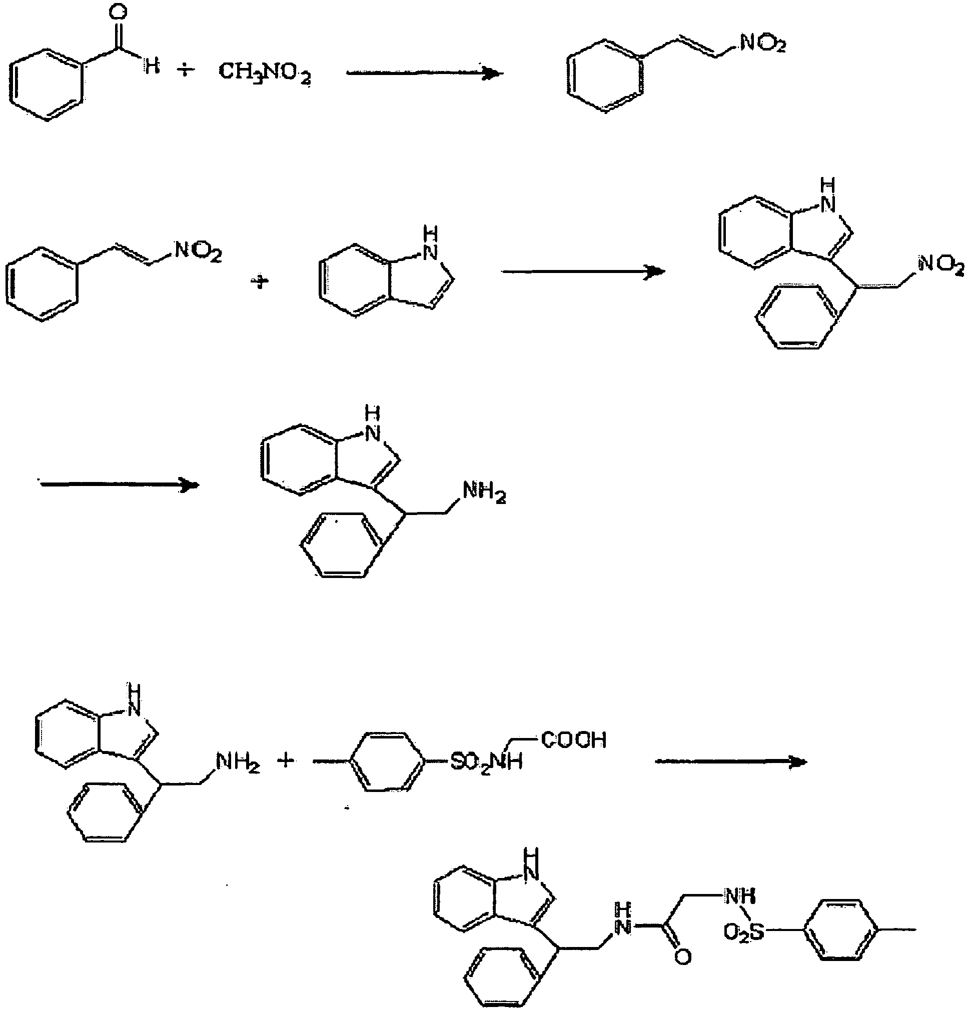

### Example 5

The effect of the above identified compounds on anti-tumor activity in human carcinoma cells was determined by the MTT survival assay. The MTT assay is a commonly used method in evaluation of cell survival, based on the ability of viable cells to convert MTT (MTT, Sigma, Cat No. 044K5307), a soluble tetrazolium salt [3-(4,5-dimethylthuazole-2-yl)-2,5 diphenyl tetrazolium bromide], into an insoluble formazan precipitate, which is quantitated by spectrophotometry following solubilization in dimethyl sulfoxide (DMSO).

In brief, different carcinoma cells were treated with negative control, various test articles at different concentration, and positive control (DDP), in 96-well tissue culture dishes. Each test article at different concentration is repeated 3 times.

Different carcinoma cells were diluted with 10% FBS and RPMI 640 or MEM media to a concentration of 3-5 x 10<4> per ml suspension. 100 microliter of cells were incubated in each well in 96-well tissue culture dishes at 37 C. The cells were incubated at 37C on the first day. On the second day, media supernatant was removed and replaced with media and different concentration of test articles (100 microliter in each well). Three Negative control wells and three positive control (DDP) wells were also included. The 96-well plate were incubated at 37 C for another 68 hours. After 68 hours, supernatant of each well was removed. 100 microliter of 1 mg/ml MTT diluted in PBS was injected into each well. The cells were incubated at 37C for another 4 hours. The cells were then solubilized in 150 microliter DMSO, mixed for 10 minutes and absorbance readings were taken using Microplate reader Bio-Rad Model 550 at 570 nm. IR (cell growth inhibition rate)% was calculated as (OD570 of negative control - OD570 with test article)/OD570 of negative control. IC50 (cell 50% inhibition concentration) at 95% confidence level was calculated with Bliss method.

### Cervical Cancer Line (Hela Cells) IC50 Results

| *Test Article* | Concentration (µg/ml) | OD(A570nm) | IR (%) | IC₅₀ (95% confidence level) |
|---|---|---|---|---|
| *Negative Control* | 0 | 0.743±0.045 | | |
| *Mol 44* | 16.67 | 0.688±0.045 | 7.4 | N/A |
| | 5.56 | 0.671±.0.044 | 9.7 | |
| | 1.85 | 0.737±0.113 | 0.8 | |
| | 0.62 | 0.765±0.200 | - | |
| | 0.21 | 0.655±0.007 | 11.8 | |
| | 0.07 | 0.599±0.109 | 19.3 | |
| *Mol 11* | 100 | 0.145±0.031 | 80.5 | 31.85 26.78-38.33 |
| | 33.33 | 0.283±0.047 | 61.9 | |
| | 11.11 | 0.653±0.134 | 12.0 | |
| | 3.70 | 0.723±0.032 | 2.6 | |
| | 1.23 | 0.790±0.037 | - | |
| | 0.41 | 0.738±0.080 | 0.7 | |
| *Mol 21* | 100 | 0.130±0.039 | 82.5 | 22.23 18.80-26.44 |
| | 33.33 | 0.194±0.031 | 73.8 | |
| | 11.11 | 0.480±0.035 | 35.4 | |
| | 3.70 | 0.774±0.066 | - | |
| | 1.23 | 0.785±0.081 | - | |
| | 0.41 | 0.668±0.049 | 10.0 | |
| *Mol 640* | 100 | 0.078±0.023 | 89.5 | 25.26 21.94-29.12 |
| | 33.33 | 0.172±0.010 | 76.8 | |
| | 11.11 | 0.652±0.057 | 12.2 | |
| | 3.70 | 0.819±0.085 | - | |
| | 1.23 | 0.826±0.101 | - | |
| | 0.41 | 0.651±0.044 | 12.3 | |
| DDP (cisplatin) (positive control) | 4 | 0.078±0.001 | 89.5 | 0.105 0.062-0.156 |
| | 1 | 0.123±0.007 | 83.4 | |
| | 0.25 | 0.206±0.014 | 72.2 | |
| | 0.0625 | 0.481±0.081 | 35.3 | |

### Breast Cancer Cell Line (MCF-7) IC50 Results

| *Test Article* | Concentration (µg/ml) | OD(A570nm) | IR (%) | IC₅₀ (95% confidence level) |
|---|---|---|---|---|
| *Negative Control* | 0 | 0.903±0.083 | | |
| *Mol 44* | 50 | 0.527±0.035 | 41.6 | 145.21 71.33-551.08 |
| | 25 | 0.587±0.030 | 34.9 | |
| | 12.5 | 0.791±0.151 | 12.4 | |
| | 6.25 | 0.791±0.151 | 10.2 | |
| | 3.125 | 0.811±0.109 | 12.8 | |
| | 1.56 | 0.787±0.176 | 13.3 | |
| *Mol 11* | 100 | 0.478±0.079 | 47.0 | 94.23 66.90-154.16 |
| | 50 | 0.490±0.093 | 45.7 | |
| | 25 | 0.705±0.076 | 21.9 | |
| | 12.5 | 0.752±0.164 | 16.6 | |
| | 6.25 | 0.711±0.045 | 21.2 | |
| | 3.125 | 0.903±0.194 | 0 | |
| *Mol* 21 | 100 | 0.072±0.007 | 92.1 | 9.93 8.53-11.45 |
| | 50 | 0.069±0.006 | 92.4 | |
| | 25 | 0.098±0.006 | 89.1 | |
| | 12.5 | 0.346±0.022 | 61.6 | |
| | 6.25 | 0.624±0.008 | 30.9 | |
| | 3.125 | 0.789±0.064 | 12.5 | |
| *Mol 640* | 100 | 0.069±0.007 | 92.3 | 14.12 12.34-16.11 |
| | 50 | 0.098±0.012 | 89.1 | |
| | 25 | 0.094±0.012 | 89.6 | |
| | 12.5 | 0.649±0.036 | 28.1 | |
| | 6.25 | 0.708±0.058 | 21.5 | |
| | 3.125 | 0.807±0.016 | 10.5 | |
| DDP (cisplatin) (positive control) | 4 | 0.107±0.007 | 88.2 | 0.596 0.483-0.737 |
| | 1 | 0.151±0.011 | 83.3 | |
| | 0.25 | 0.879±0.059 | 2.6 | |
| | 0.0625 | 0.787±0.005 | 12.8 | |

### Colon Cancer Cell Line (HCT116) IC50 Results

| *Test Article* | Concentration (µg/ml) | OD(A570nm) | IR (%) | IC₅₀ (95% confidence level) |
|---|---|---|---|---|
| *Negative Control* | 0 | 0.542±0.027 | | |
| *Mol 21* | 100 | 0.083±0.001 | 84.7 | 3.160 2.992-3.330 |
| | 50 | 0.085±0.004 | 84.3 | |
| | 25 | 0.101±0.020 | 81.3 | |
| | 12.5 | 0.130±0.025 | 72.3 | |
| | 6.25 | 0.300±0.050 | 44.6 | |
| | 3.125 | 0.237±0.098 | 56.2 | |
| | 100 | 0.086±0.012 | 84.1 | |
| | 50 | 0.083±0.011 | 84.6 | |
| | 25 | 0.109±0.006 | 80.0 | |
| | 12.5 | 0.267±0.035 | 50.7 | |
| | 6.25 | 0.394±0.039 | 27.3 | |
| | 3.125 | 0.377±0.025 | 30.4 | |
| DDP | 4 | 0.133±0.016 | 75.5 | 0.234 0.211-0.257 |
| | 1 | 0.201±0.060 | 62.9 | |
| | 0.25 | 0.266±0.016 | 51.0 | |

### Lung Cancer Cell Line (A459) IC50 Results

| *Test Article* | Concentration (µg/ml) | OD(A570nm) | IR (%) | IC₅₀ (95% confidence level) |
|---|---|---|---|---|
| *Negative Control* | 0 | 0.637±0.044 | | |
| *Mol 44* | 50 | 0.368±0.039 | 42.3 | 150 |
| | 25 | 0.504±0.046 | 21.0 | |
| | 12.5 | 0.586±0.051 | 8.1 | |
| | 6.25 | 0.680±0.01.5 | -6.8 | |
| | 3.125 | 0.786±0.048 | -23.3 | |
| | 1.56 | 0.634±0.009 | 0.6 | |
| *Mol 11* | 100 | 0.108±0.013 | 83.1 | 19.435 19.116-19.759 |
| | 50 | 0.152±0.017 | 76.1 | |
| | 25 | 0.230±0.040 | 64.0 | |
| | 12.5 | 0.438±0.051 | 31.2 | |
| | 6.25 | 0.471±0.001 | 26.1 | |
| | 3.125 | 0.583±0.015 | 8.6 | |
| DDP | 4 | 0.185±0.231 | 71.0 | 1.162 1.113-1.213 |
| | 1 | 0.341±0.060 | 46.4 | |
| | 0.25 | 0.517±0.073 | 18.8 | |
| | 0.0625 | 0.483±0.046 | 23.9 | |

### Lung Cancer A459 Cell Line Experiment #2

| *Test Article* | Concentration (µg/ml) | OD(A570nm) | IR (%) | IC₅₀ (95% confidence level) |
|---|---|---|---|---|
| *Negative Control* | 0 | 0.637±0.044 | | |
| *Mol 21* | 100 | 0.082±0.015 | 87.1 | 6.720 6.303-7.134 |
| | 50 | 0.069±0.010 | 89.2 | |
| | 25 | 0.118±0.027 | 81.5 | |
| | 12.5 | 0.279±0.014 | 56.2 | |
| | 6.25 | 0.662±0.031 | -3.9 | |
| | 3.125 | 0.642±0.073 | -0.7 | |
| *Mol 640* | 100 | 0.082±0.014 | 87.1 | 25.828 25.516-26.141 |
| | 50 | 0.081±0.010 | 87.2 | |
| | 25 | 0.232±0.006 | 63.6 | |
| | 12.5 | 0.593±0.034 | 6.9 | |
| | 6.25 | 0.717±0.020 | -12.5 | |
| | 3.125 | 0.781±0.040 | -22.6 | |
| DDP | 4 | 0.185±0.231 | 71.0 | 1.162 |
| | 1 | 0.341±0.060 | 46.4 | 1.114-1.213 |
| | 0.25 | 0.517±0.073 | 18.8 | |
| | 0.0625 | 0.485±0.046 | 23.9 | |

### Liver Cancer Cell Line (SMMC-7721) IC50 Results

| *Test Article* | Concentration (µg/ml) | OD(A570nm) | IR (%) | IC₅₀ (95% confidence level) |
|---|---|---|---|---|
| *Negative Control* | 0 | 0.440±0.049 | | |
| *Mol 44* | 50 | 0.345±0.025 | 21.6 | |
| | 25 | 0.373±0.019 | 15.4 | |
| | 12.5 | 0.392±0.039 | 11.1 | |
| | 6.25 | 0.272±0.019 | 38.3 | |
| | 3.125 | 0.321±0.088 | 27.2 | |
| | 1.56 | 0.275±0.004 | 37.6 | |
| *Mol 11* | 100 | 0.141±0.006 | 68.1 | 14.934 14.338-15.549 |
| | 50 | 0.163±0.009 | 63.0 | |
| | 25 | 0.205±0.018 | 53.5 | |
| | 12.5 | 0.207±0.007 | 53.1 | |
| | 6.25 | 0.286±0.092 | 35.1 | |
| | 3.125 | 0.277±0.018 | 37.0 | |
| DDP | 4 | 0.169±0.022 | 61.6 | |
| | 1 | 0.128±0.011 | 71.0 | |
| | 0.25 | 0.237±0.020 | 46.2 | |
| | 0.0625 | 0.570±0.166 | -29.4 | |

### Liver Cancer SMMC-7721 Cell Line Experiment #2

| *Test Article* | Concentration (µg/ml) | OD(A570nm) | IR (%) | IC₅₀ (95% confidence level) |
|---|---|---|---|---|
| *Negative Control* | 0 | 0.461±0.271 | | |
| | | | | |
| *Mol 21* | 100 | 0.077±0.009 | 83.2 | |
| | 50 | 0.079±0.005 | 82.9 | |
| | 25 | 0.129±0.006 | 72.0 | 9.515 |
| | 12.5 | 0.146±0.041 | 68.3 | 9.321-9.712 |
| | 6.25 | 0.161±0.085 | 65.1 | |
| | 3.125 | 0.690±0.603 | -49.9 | |
| | | | | |
| | | 0.098±0.014 | 78.7 | |
| *Mol 640* | 100 | | | |
| | 50 | 0.091±0.002 | 80.2 | 8.018 |
| | 25 | 0.200±0.002 | 56.6 | 7.681-8.359 |
| | 12.5 | 0.268±0.013 | 41.9 | |
| | 6.25 | 0.278±0.013 | 39.8 | |
| | 3.125 | 0.215±0.008 | 53.4 | |
| | | | | |
| DDP | 4 | 0.244±0.155 | 47.0 | 4.155 |
| | 1 | 0.262±0.020 | 43.1 | 3.757-4.640 |
| | 0.25 | 0.348±0.045 | 24.5 | |

### Ovarian Cancer Cell Line (SKOV3) IC50 Results

| *Test Article* | Concentration (µg/ml) | OD(A570nm) | IR (%) | IC₅₀ (95% confidence level) |
|---|---|---|---|---|
| *Negative control* | 0 | 0.701±0.03.5 | | |
| *Mol44* | 50 | 0.333±0.008 | 52.4 | 60.00 |
| | 25 | 0.426±0.008 | 39.1 | |
| | 12.5 | 0.525±0.035 | 25.1 | |
| | 6.25 | 0.539±0.023 | 23.0 | |
| | 3.125 | 0.533±0.017 | 23.9 | |
| | 1.56 | 0.505±0.029 | 27.9 | |
| *Mol11* | 100 | 0.098±0.010 | 86.0 | 29.00 |
| | 50 | 0.314±0.041 | 55.1 | |
| | 25 | 0.385±0.017 | 45.0 | |
| | 12.5 | 0.671±0.063 | 4.1 | |
| | 6.25 | 0.694±0.043 | 0.9 | |
| | 3.125 | 0.753±0.074 | 0 | |
| DDP | 4 | 0.083±0.002 | 88.2 | 0.89 |
| | 1 | 0.267±0.044 | 62.0 | |
| | 0.25 | 0.643±0.141 | 8.3 | |
| | 0.0625 | 0.689±0.065 | 1.6 | |

### Ovarian Cancer Cell Line (SKOV3) IC50 Results

| *Test Article* | Concentration (µg/ml) | OD(A570nm) | IR (%) | IC₅₀ (95% confidence level) |
|---|---|---|---|---|
| *Negative control* | 0 | 0.701±0.035 | | |
| *Mol21* | 100 | 0.077±0.011 | 89.1 | 17.69 |
| | 50 | 0.119±0.010 | 83.0 | |
| | 25 | 0.429±0.002 | 38.8 | |
| | 12.5 | 0.460±0.195 | 34.3 | |
| | 6.25 | 0.532±0.025 | 24.1 | |
| | 3.125 | 0.503±0.031 | 28.2 | |
| *Mol640* | 100 | 0.079±0.010 | 88.7 | 26.87 |
| | 50 | 0.082±0.008 | 88.3 | |
| | 25 | 0.394±0.037 | 43.8 | |
| | 12.5 | 0.632±0.020 | 9.7 | |
| | 6.25 | 0.628±0.025 | 10.3 | |
| | 3.125 | 0.716±0.048 | 0 | |
| DDP | 4 | 0.083±0.002 | 88.2 | 0.89 |
| | 1 | 0.267±0.044 | 62.0 | |
| | 0.25 | 0.643±0.141 | 8.3 | |
| | 0.0625 | 0.689±0.065 | 1.6 | |

### Glioma Cell Line (Brain Tumor U251) IC50 Results

| *Test Article* | Concentration (µg/ml) | OD(A570nm) | IR (%) | IC₅₀ (95% confidence level) |
|---|---|---|---|---|
| *Negative control* | 0 | 0.877±0.055 | | |
| *Mol44* | 50 | 0.659±0.047 | 24.9 | 129.14 |
| | 25 | 0.737±0.067 | 15.9 | |
| | 12.5 | 0.811±0.050 | 7.5 | |
| | 6.25 | 0.866±0.090 | 1.2 | |
| | 3.125 | 0.705±0.061 | 19.6 | |
| | 1.56 | 0.790±0.086 | 9.9 | |
| *Mol11* | 100 | 0.650±0.006 | 25.8 | 4340 |
| | 50 | 0.709±0.054 | 19.1 | |
| | 25 | 0.697±0.053 | 20.5 | |
| | 12.5 | 0.744±0.045 | 15.1 | |
| | 6.25 | 0.758±0.019 | 13.5 | |
| | 3.125 | 0.797±0.016 | 9.1 | |
| DDP | 4 | 0.174±0.009 | 80.2 | 1.13 |
| | 1 | 0.365±0.058 | 58.4 | |
| | 0.25 | 0.688±0.035 | 21.5 | |
| | 0.0625 | 0.729±0.090 | 16.8 | |

### Glioma Cell Line (Brain Tumor U251) IC50 Results

| *Test Article* | Concentration (µg/ml) | OD(A570nm) | IR (%) | IC₅₀ (95% confidence level) |
|---|---|---|---|---|
| *Negative control* | 0 | 0.877±0.055 | | |
| *Mol21* | 100 | 0.094±0.007 | 89.2 | 16.24 |
| | 50 | 0.121±0.019 | 86.2 | |
| | 25 | 0.232±0.034 | 73.5 | |
| | 12.5 | 0.501±0.058 | 42.8 | |
| | 6.25 | 0.719±0.110 | 18.0 | |
| | 3.125 | 0.840±0.013 | 4.2 | |
| *Mol640* | 100 | 0.090±0.014 | 89.7 | 11.65 |
| | 50 | 0.087±0.012 | 90.1 | |
| | 25 | 0.085±0.012 | 90.3 | |
| | 12.5 | 0.354±0.021 | 59.6 | |
| | 6.25 | 0.696±0.054 | 20.6 | |
| | 3.125 | 0.796±0.055 | 9.2 | |
| DDP | 4 | 0.174±0.009 | 80.2 | 1.13 |
| | 1 | 0.365±0.058 | 58.4 | |
| | 0.25 | 0.688±0.035 | 21.5 | |
| | 0.0625 | 0.729±0.090 | 16.8 | |

All patents, patent applications, and literature references referred to herein are hereby incorporated by reference in their entirety. Many variations of the present invention will suggest themselves to those skilled in the art in light of the above detailed description. Such obvious variations are within the full-intended scope of the appended claims.

Note: E3 binding pocket for design is surrounded by the following residues Val634, Leu635, Ser638, Leu639, Leu642, Met 653, Ile655, Ile659, Ile682, Phe690, Tyr694, in E6AP 3-D structure in Huang et. al.. In full length E6AP (or UBE3A_human), these residues are Val657, Leu658, Ser661, Leu662, Leu665, Met 676, Ile678, Ile682, Ile705, Phe713, Tyr717.

### REFERENCES CITED

Balasubramanyam M. et al. Mol. Cell Biochem. 275(1-2), 117-25 (2005)
Bueso-Ramos, CE. et al. Blood 82, 2617-2623 (1993).
Fuchs, S.Y., Adler, V., Buschmann, T., Wu, X. & Ronai, Z. Oncogene 17, 2543-2547 (1998).
Gustafsson, B. & Stal, O. Pediatr Hematol Oncol 15, 519-526 (1998).
Hall, EJ. Int J Radiat Oncol Biol Phys 30, 1019-1028 (1994).
Hochstrasser, M. Annu Rev Genet 30, 405-439 (1996).
Honda, R., Tanaka, H. & Yasuda, H. FEBS Lett 420, 25-27 (1997).
Huang, L. et al. Science 286, 1321-1326 (1999).
Keating, M.J. Clin Cancer Res 3, 2598-2604 (1997).
Konstantinopoulos PA et al. Expert Opin Investig Drugs 15(9), 1067-75 (2006)
Magrath, I. Cancer Res 52, 5529s-5540s (1992).
Masdehors, P. et al. Blood 96, 269-274 (2000).
Montagut C. et al. Clin Transl Oncol. 8(5): 313-7 (2006)
Oren, M. Semin Cancer Biol 5, 221-227 (1994).
Pan, Y. & Haines, D.S. Cancer Res 59, 2064-2067 (1999).
Pulczynski, S. Leuk Lymphoma 15, 243-252 (1994).
Scheffner, M., Smith, S. & Jentsch, S. in Ubiquitin and the Biology of the Cell, Plenum, NY,
Peters, J.-M., Harris, J. R., and Finley, D., 65-98 (1998).
Schwarz, S.E., Rosa, JX. & Scheffner, M. J Biol Chem 273, 12148-12154 (1998).
Soussi, T. & Jonveaux, P. Nouv Rev Fr Hematol 33, 477-480 (1991).
Starita LM. et al. Cancer Biol Ther 5(2), 137-141 (2006)
Teoh, G et al. Blood 90, 1982-1992 (1997).
Voutsadakis, LA. Acta Oncol 39, 151-156 (2000).
Williams AJ et al. Neurochem Int. 49(2), 106-12 (2006)
Zheng, N. et al. Cell 102, 533-539 (2000)

## Claims

1. A E2-E3 inhibitor with anti-tumor activity for use in therapy, which is a compound having the following formula I : 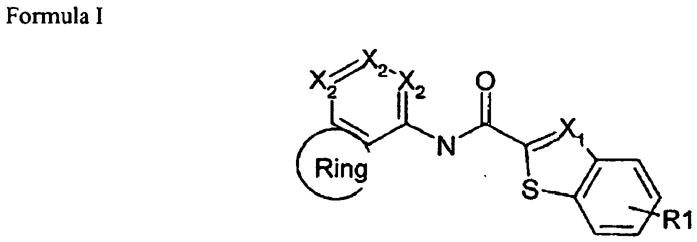
X1 = N, C
R1 = halogens (F, Cl, Br), Me, OMe, OH
mono- or multi- substituted at the free valences using combinations of the functional groups
X2 = one of more of it could be either C or N
Ring = five- or six- membered rings fuzed with the aromatic ring explicitly drawn, including benzene, pyridine, pyrimidine, pyrizine, pyrrole, imidazole, furan, oxazole

2. A pharmaceutical composition comprising at least one compound of claim 1 and a pharmaceutical carrier.

3. Use of the inhibitor of claim 1 for the manufacture of a medicament for the treatment of cancer.

4. The use of claim 3, wherein the cancer is brain tumor, lung cancer, ovarian cancer, bladder cancer, cervical cancer, colon cancer, breast cancer, and prostate cancer.

5. The use of claim 3 or 4, wherein the treatment comprises: administering a therapeutically effective amount of at least one compound of claim 1 to a subject in need thereof.

6. The use of claim 5, wherein the treatment further comprises the step of administering a therapeutically effective amount of a chemotherapy agent within the therapeutic window for this chemotherapy agent to said patient to achieve a therapeutically effective change in progression of the cancer selected from the group consisting of brain tumor, lung cancer, ovarian cancer, bladder cancer, cervical cancer, colon cancer, breast cancer, and prostate cancer.

7. The use of claim 5, wherein the treatment further comprises the step of administering a therapeutically dose of radiotherapy within the therapeutic window for this radiotherapy to said patient to achieve a therapeutically effective change in progression of said cancer selected from the group consisting of brain tumor, lung cancer, ovarian cancer, bladder cancer, cervical cancer, colon cancer, breast cancer, and prostate cancer.

8. The use of any one of claims 5-7, wherein the compound of claim 1 is combined with other chemotherapy agents.

9. Use of the inhibitor of claim 1 for the manufacture of an imaging agent for tumor diagnosis.

## Patentansprüche

1. E2-E3-Inhibitor mit Antitumoraktivität zur Verwendung in der Therapie, wobei der Inhibitor eine Verbindung ist, welche die folgende Formel I aufweist: 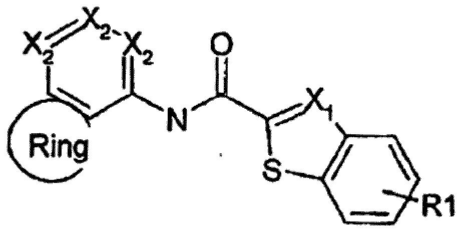
X1 = N, C
R1 = Halogene (F, Cl, Br), Me, OMe, OH
einfach oder mehrfach an den freien Valenzen substituiert mittels Kombinationen der funktionellen Gruppen
X2 = eins oder mehrere könnten entweder C oder N sein
Ring = fünf- oder sechsgliedrige Ringe, verschmolzen mit dem ausdrücklich gezeichneten aromatischen Ring, einschließlich Benzol, Pyridin, Pyrimidin, Pyrizin, Pyrrol, Imidazol, Furan, Oxazol.

2. Pharmazeutische Zusammensetzung, umfassend mindestens eine Verbindung nach Anspruch 1 und einen pharmazeutischen Träger.

3. Verwendung des Inhibitors nach Anspruch 1 zur Herstellung eines Medikaments für die Behandlung von Krebs.

4. Verwendung nach Anspruch 3, wobei der Krebs Hirntumor, Lungenkrebs, Eierstockkrebs, Blasenkrebs, Gebärmutterhalskrebs, Darmkrebs, Brustkrebs, und Prostatakrebs ist.

5. Verwendung nach Anspruch 3 oder 4, wobei die Behandlung umfasst: Verabreichung einer therapeutisch wirksamen Menge von mindestens einer Verbindung nach Anspruch 1 an eine Testperson, die diese benötigt.

6. Verwendung nach Anspruch 5, wobei die Behandlung ferner den Schritt der Verabreichung einer therapeutisch wirksamen Menge eines chemotherapeutischen Mittels innerhalb des therapeutischen Fensters für dieses chemotherapeutische Mittel an die Testperson umfasst, um eine therapeutisch wirksame Veränderung im Verlauf des Krebs zu erzielen, wobei der Krebs ausgewählt ist aus der Gruppe bestehend aus Gehirntumor, Lungenkrebs, Eierstockkrebs, Blasenkrebs, Gebärmutterhalskrebs, Darmkrebs, Brustkrebs, und Prostatakrebs.

7. Verwendung nach Anspruch 5, wobei die Behandlung ferner den Schritt der Verabreichung einer therapeutischen Bestrahlungsdosis innerhalb des therapeutischen Fensters für diese Bestrahlung an die Testperson umfasst, um eine therapeutisch wirksame Veränderung im Verlauf des Krebs zu erzielen, wobei der Krebs ausgewählt ist aus der Gruppe bestehend aus Hirntumor, Lungenkrebs, Eierstockkrebs, Blasenkrebs, Gebärmutterhalskrebs, Darmkrebs, Brustkrebs, und Prostatakrebs.

8. Verwendung nach einem der Ansprüche 5-7, wobei die Verbindung nach Anspruch 1 mit anderen chemotherapeutischen Mitteln kombiniert wird.

9. Verwendung des Inhibitors nach Anspruch 1 zur Herstellung eines Bildgebungsmittels für die Tumordiagnostik.

## Revendications

1. Inhibiteur de E2-E3 avec une activité anticancéreuse pour utilisation en thérapie, qui est un composé ayant la formule I suivante : 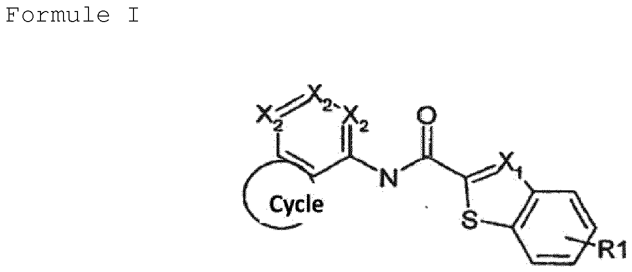
X1 = N, C
R1 = halogènes (F, Cl, Br), Me, OMe, OH
mono- ou multi-substitués aux valences libres en utilisant des combinaisons des groupes fonctionnels
X2 = un ou plusieurs de celui-ci peuvent être C ou N Cycle = cycles de cinq ou six chaînons fusionnés avec le cycle aromatique explicitement dessiné, comprenant le benzène, la pyridine, la pyrimidine, la pyrizine, le pyrrole, l'imidazole, le furane, l'oxazole.

2. Composition pharmaceutique comprenant au moins un composé de la revendication 1 et un véhicule pharmaceutique.

3. Utilisation de l'inhibiteur de la revendication 1 pour la fabrication d'un médicament pour le traitement du cancer.

4. Utilisation de la revendication 3, dans laquelle le cancer est une tumeur du cerveau, le cancer du poumon, le cancer ovarien, le cancer de la vessie, le cancer cervical, le cancer du sein, et le cancer de la prostate.

5. Utilisation de la revendication 3 ou 4, dans laquelle le traitement comprend : l'administration d'une quantité thérapeutiquement efficace d'au moins un composé de la revendication 1 à un sujet nécessitant celle-ci.

6. Utilisation de la revendication 5, dans laquelle le traitement comprend en outre l'étape d'administration d'une quantité thérapeutiquement efficace d'un agent de chimiothérapie dans la fenêtre thérapeutique pour cet agent de chimiothérapie audit patient pour obtenir un changement thérapeutiquement efficace de la progression du cancer choisi dans le groupe constitué d'une tumeur du cerveau, du cancer du poumon, du cancer ovarien, du cancer de la vessie, du cancer cervical, du cancer du sein, et du cancer de la prostate.

7. Utilisation de la revendication 5, dans laquelle le traitement comprend en outre l'étape d'administration d'une dose thérapeutique de radiothérapie dans la fenêtre thérapeutique pour cette radiothérapie audit patient pour obtenir un changement thérapeutiquement efficace de la progression dudit cancer choisi dans le groupe constitué d'une tumeur du cerveau, du cancer du poumon, du cancer ovarien, du cancer de la vessie, du cancer cervical, du cancer du sein, et du cancer de la prostate.

8. Utilisation de l'une quelconque des revendications 5 à 7, dans laquelle le composé de la revendication 1 est combiné avec d'autres agents de chimiothérapie.

9. Utilisation de l'inhibiteur de la revendication 1 pour la fabrication d'un agent d'imagerie pour un diagnostic de tumeur.


### SEQUENCE LISTING

<110> Huang, Lan
   xing, Li
<120> New binding pocket for ubiquitin ligase E3, its use in designing E2 inhibitors, the process for designing E2 inhibitors by the use of said pocket, the inhibitors designed by said process and the use of said inhibitors for cancer treatment
<130>
<150> us60/856,125
   <151> 2006-11-02
<160> 1
<170> PatentIn version 3.4
<210> 1
   <211> 875
   <212> PRT
   <213> UBE3A_human
<400> 1
   Met Glu Lys Leu His Gln Cys Tyr Trp Lys Ser Gly Glu Pro Gln Ser
   1               5                   10                  15
   Asp Asp Ile Glu Ala Ser Arg Met Lys Arg Ala Ala Ala Lys His Leu
               20                  25                  30
   Ile Glu Arg Tyr Tyr His Gln Leu Thr Glu Gly Cys Gly Asn Glu Ala
           35                  40                  45
   Cys Thr Asn Glu Phe Cys Ala Ser Cys Pro Thr Phe Leu Arg Met Asp
       50                  55                  60
   Asn Asn Ala Ala Ala Ile Lys Ala Leu Glu Leu Tyr Lys Ile Asn Ala
   65                  70                  75                  80
   Lys Leu Cys Asp Pro His Pro Ser Lys Lys Gly Ala Ser Ser Ala Tyr
                   85                  90                  95
   Leu Glu Asn Ser Lys Gly Ala Pro Asn Asn Ser Cys Ser Glu Ile Lys
               100                 105                 110
   Met Asn Lys Lys Gly Ala Arg Ile Asp Phe Lys Asp Val Thr Tyr Leu
           115                 120                 125
   Thr Glu Glu Lys Val Tyr Glu Ile Leu Glu Leu Cys Arg Glu Arg Glu
       130                 135                 140
   Asp Tyr Ser Pro Leu Ile Arg Val Ile Gly Arg Val Phe Ser Ser Ala
   145                 150                 155                 160
   Glu Ala Leu Val Gln Ser Phe Arg Lys Val Lys Gln His Thr Lys Glu
                   165                 170                 175
   Glu Leu Lys Ser Leu Gln Ala Lys Asp Glu Asp Lys Asp Glu Asp Glu
               180                 185                 190
   Lys Glu Lys Ala Ala Cys Ser Ala Ala Ala Met Glu Glu Asp Ser Glu
           195                 200                 205
   Ala Ser Ser Ser Arg Ile Gly Asp Ser Ser Gln Gly Asp Asn Asn Leu
       210                 215                 220
   Gln Lys Leu Gly Pro Asp Asp Val Ser Val Asp Ile Asp Ala Ile Arg
   225                 230                 235                 240
   Arg Val Tyr Thr Arg Leu Leu Ser Asn Glu Lys Ile Glu Thr Ala Phe
                   245                 250                 255
   Leu Asn Ala Leu Val Tyr Leu Ser Pro Asn Val Glu Cys Asp Leu Thr
               260                 265                 270
   Tyr His Asn Val Tyr Ser Arg Asp Pro Asn Tyr Leu Asn Leu Phe Ile
           275                 280                 285
   Ile Gly Met Glu Asn Arg Asn Leu His Ser Pro Glu Tyr Leu Glu Met
       290                 295                 300
   Ala Leu Pro Leu Phe Cys Lys Ala Met Ser Lys Leu Pro Leu Ala Ala
   305                 310                 315                 320
   Gln Gly Lys Leu Ile Arg Leu Trp Ser Lys Tyr Asn Ala Asp Gln Ile
                   325                 330                 335
   Arg Arg Met Met Glu Thr Phe Gln Gln Leu Ile Thr Tyr Lys Val Ile
               340                 345                 350
   Ser Asn Glu Phe Asn Ser Arg Asn Leu Val Asn Asp Asp Asp Ala Ile
           355                 360                 365
   Val Ala Ala Ser Lys Cys Leu Lys Met Val Tyr Tyr Ala Asn Val Val
       370                 375                 380
   Gly Gly Glu Val Asp Thr Asn His Asn Glu Glu Asp Asp Glu Glu Pro
   385                 390                 395                 400
   Ile Pro Glu Ser Ser Glu Leu Thr Leu Gln Glu Leu Leu Gly Glu Glu
                   405                 410                 415
   Arg Arg Asn Lys Lys Gly Pro Arg Val Asp Pro Leu Glu Thr Glu Leu
               420                 425                 430
   Gly Val Lys Thr Leu Asp Cys Arg Lys Pro Leu Ile Pro Phe Glu Glu
           435                 440                 445
   Phe Ile Asn Glu Pro Leu Asn Glu Val Leu Glu Met Asp Lys Asp Tyr
       450                 455                 460
   Thr Phe Phe Lys Val Glu Thr Glu Asn Lys Phe Ser Phe Met Thr Cys
   465                 470                 475                 480
   Pro Phe Ile Leu Asn Ala Val Thr Lys Asn Leu Gly Leu Tyr Tyr Asp
                   485                 490                 495
   Asn Arg Ile Arg Met Tyr Ser Glu Arg Arg Ile Thr Val Leu Tyr Ser
               500                 505                 510
   Leu Val Gln Gly Gln Gln Leu Asn Pro Tyr Leu Arg Leu Lys Val Arg
           515                 520                 525
   Arg Asp His Ile Ile Asp Asp Ala Leu Val Arg Leu Glu Met Ile Ala
       530                 535                 540
   Met Glu Asn Pro Ala Asp Leu Lys Lys Gln Leu Tyr Val Glu Phe Glu
   545                 550                 555                 560
   Gly Glu Gln Gly Val Asp Glu Gly Gly Val Ser Lys Glu Phe Phe Gln
                   565                 570                 575
   Leu Val Val Glu Glu Ile Phe Asn Pro Asp Ile Gly Met Phe Thr Tyr
               580                 585                 590
   Asp Glu Ser Thr Lys Leu Phe Trp Phe Asn Pro Ser Ser Phe Glu Thr
           595                 600                 605
   Glu Gly Gln Phe Thr Leu Ile Gly Ile Val Leu Gly Leu Ala Ile Tyr
       610                 615                 620
   Asn Asn Cys Ile Leu Asp Val His Phe Pro Met Val Val Tyr Arg Lys
   625                 630                 635                 640
   Leu Met Gly Lys Lys Gly Thr Phe Arg Asp Leu Gly Asp Ser His Pro
                   645                 650                 655
   Val Leu Tyr Gln Ser Leu Lys Asp Leu Leu Glu Tyr Glu Gly Asn Val
               660                 665                 670
   Glu Asp Asp Met Met Ile Thr Phe Gln Ile Ser Gln Thr Asp Leu Phe
           675                 680                 685
   Gly Asn Pro Met Met Tyr Asp Leu Lys Glu Asn Gly Asp Lys Ile Pro
       690                 695                 700
   Ile Thr Asn Glu Asn Arg Lys Glu Phe Val Asn Leu Tyr Ser Asp Tyr
   705                 710                 715                 720
   Ile Leu Asn Lys Ser Val Glu Lys Gln Phe Lys Ala Phe Arg Arg Gly
                   725                 730                 735
   Phe His Met Val Thr Asn Glu Ser Pro Leu Lys Tyr Leu Phe Arg Pro
               740                 745                 750
   Glu Glu Ile Glu Leu Leu Ile Cys Gly Ser Arg Asn Leu Asp Phe Gln
           755                 760                 765
   Ala Leu Glu Glu Thr Thr Glu Tyr Asp Gly Gly Tyr Thr Arg Asp Ser
       770                 775                 780
   Val Leu Ile Arg Glu Phe Trp Glu Ile Val His Ser Phe Thr Asp Glu
   785                 790                 795                 800
   Gln Lys Arg Leu Phe Leu Gln Phe Thr Thr Gly Thr Asp Arg Ala Pro
                   805                 810                 815
   Val Gly Gly Leu Gly Lys Leu Lys Met Ile Ile Ala Lys Asn Gly Pro
               820                 825                 830
   Asp Thr Glu Arg Leu Pro Thr Ser His Thr Cys Phe Asn Val Leu Leu
           835                 840                 845
   Leu Pro Glu Tyr Ser Ser Lys Glu Lys Leu Lys Glu Arg Leu Leu Lys
       850                 855                 860
   Ala Ile Thr Tyr Ala Lys Gly Phe Gly Met Leu
   865                 870                 875